(19) Europäisches Patentamt
European Patent Office
Office européen des brevets

(11) Numéro de publication: **0 258 134 B1**

# (12) FASCICULE DE BREVET EUROPEEN

(45) Date de publication du fascicule du brevet: **23.05.90**

(51) Int. Cl.⁵: **A61K 31/16,** A61K 31/15

(21) Numéro de dépôt: **87401873.2**

(22) Date de dépôt: **12.08.87**

(54) **Compsitions thérapeutiques contenant des dérivés du benzhydrylthiométhane.**

(30) Priorité: **13.08.86 FR 8611683**
**30.10.86 FR 8615129**

(43) Date de publication de la demande:
**02.03.88 Bulletin 88/9**

(45) Mention de la délivrance du brevet:
**23.05.90 Bulletin 90/21**

(84) Etats contractants désignés:
**AT BE CH DE ES FR GB GR IT LI LU NL SE**

(56) Documents cités:
**EP-A- 0 097 071**
**EP-A- 0 097 547**
**DE-A- 2 814 445**
**FR-A- 2 326 181**
**FR-A- 2 385 693**

(73) Titulaire: **LABORATOIRE L. LAFON, 19 Avenue du Professeur Cadiot, F-94701 Maisons Alfort(FR)**

(72) Inventeur: **Lafon, Louis, 5 rue de l'Alboni, F-75016 Paris(FR)**

(74) Mandataire: **Moncheny, Michel et al, c/o Cabinet Lavoix 2 Place d'Estienne d'Orves, F-75441 Paris Cedex 09(FR)**

EP 0 258 134 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des compositions thérapeutiques contenant à titre de principe actif un dérivé du benzhydrylthiométhane qui peuvent être utilisées en thérapeutique en raison de leur activité sur le système nerveux central et pour certaines en raison de leur activité immunostimulante.

On a déjà décrit un certain nombre de dérivés de benzhydrylthiométhane qui sont utilisés dans la présente invention.

Ainsi, FR-A-2 385 693 décrit le 2-(benzhydryl thio)acétamide, EP-0 097 071 décrit le 2-(4,4'-difluorobenzhydryl thio)acétamide et EP-0 097 547 décrit l'acide 2-(4,4'-difluorobenzhydrylthio)acétohydroxamique. Ces composés n'ont été toutefois décrits que comme produits intermédiaires et il n'était pas évident qu'ils présentent des propriétés intéressantes permettant leur application en thérapeutique.

La présente invention a pour objet des compositions contenant à titre de principe actif un dérivé de benzhydrylthiométhane de formule

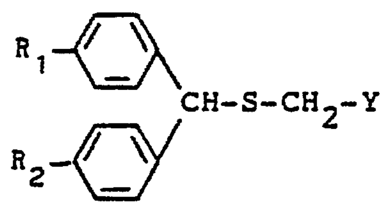

II

dans laquelle Y représente un groupe $-CONH_2$, -CO-NHOH ou -CH = N-OH et $R_1$ et $R_2$ représentent indépendamment l'un de l'autre un atome d'hydrogène ou de fluor.

Les composés de formule II dans lesquels Y = $-CONH_2$ peuvent être préparés par réaction de l'ammoniaque sur le chlorure de l'acide 2-(benzhydrylthio)acétique correspondant.

Les composés dans lesquels Y = CO-NHOH peuvent être préparés par réaction du chlorhydrate d'hydroxylamine sur l'ester méthylique de l'acide 2-(benzhydrylthio)acétique correspondant.

Les composés dans lequels Y =-CH =N-OH peuvent être préparés par réaction du chlorhydrate d'hydroxylamine sur le 2-(benzhydrylthio)acétaldéhyde correspondant.

Les exemples suivants illustrent la préparation des composés.

Exemple 1

Préparation du 2-(4,4'-difluoro benzhydryl thio)acétamide (n° du code CRL 41334)

On chauffe une heure à reflux une solution de 26,46 g (0,09 mole) d'acide 2-(4,4'-difluoro benzhydryle thio)acétique dans 145 ml de benzène et 30 ml de chlorure de thionyle.

On évapore sous vide, reprend avec 100 ml d'éther et coule dans un mélange agité de 100 ml d'ammoniaque à 28% et 200 g de glace.

On agite 2 heures, décante l'éther, lave à l'eau, sèche sur $Na_2SO_4$, évapore à sec sous vide, reprend le résidu à l'éther de pétrole et essore.

On obtient après recristallisation dans l'éther isopropylique : 18 g d'amide ($F_{inst.}$ = 74°C; Rdt. = 68%).

Le composé est une poudre blanche, insoluble dans l'éther de pétrole, soluble dans l'éther, l'acétate d'éthyle et les alcools. Sa solubilité dans l'eau est inférieure à 0,1%.

Exemple 2

Préparation de l'acide 2-(4,4'-difluoro benzhydryl thio)acétohydroxamique (n° du code CRL 41 335)

44,1 g (0,15 mole) d'acide 2-(4,4'-difluoro benzhydryl thio)acétique sont dissous dans 300 ml de méthanol et additionnés de 1,5 ml d'$H_2SO_4$ concentré. Après 4 heures à reflux, on évapore le méthanol, reprend à l'eau et à l'éther, lave au bicarbonate dilué, puis à l'eau, sèche et évapore sous vide.

Le résidu huileux est traité pendant une nuit à 20°C, avec une solution obtenu avec 6,9 g (0,3 Atg) de sodium, 10,5 g (0,15 mole) de chlorhydrate de'hydroxylamine et 480 ml de méthanol anhydre.

On évapore à sec sous vide, reprend avec 600 ml d'eau, filtre sur charbon, acidifie avec HCl 3 N, extrait à l'éther, lave à l'eau, sèche, évapore et cristallise dans l'éther de pétrole.

On recueille 28,6 g d'acide 2-(4,4'-difluoro benzhydryl thio)acétohydroxamique. ($F_{inst.}$ = 76°C; Rdt. = 62%.).

Le composé est une poudre légèrement rosée; soluble dans l'éther, l'acétate d'éthyle, les alcools; insoluble dans l'eau.

Exemple 3

Préparation du 2-(benzhydrylthio)acétamide (nº de code CRL-41055).

21 g (0,076 mole) de chlorure de benzhydrylthioacétyle, en solution dans 100 ml de chlorure de méthylène, sont ajoutés goutte à goutte en agitant dans 40 ml d'ammoniaque et 40 ml d'eau. Après une heure d'agitation, la phase organique est décantée, lavée à l'eau et séchée sur $Na_2SO_4$. On évapore le solvant sous vide, cristallise le résidu dans l'éther isopropylique et recristallise dans l'acétate d'éthyle. On obtient le composé avec un rendement de 45%.

C'est une poudre blanche, soluble dans les alcools, l'acétone, l'acétate d'éthyle; insoluble dans l'eau, l'éther isopropylique.

Il fond à 107 - 108°C.

Exemple 4

Préparation de la 2-(benzhydrylthio)acétaldoxime (nº de code CRL-40956).

a) Préparation du 2-(benzhydrylthio)acétaldéhyde.

Dans une solution de 3 g (0,13 Atg) de sodium dans 250 ml d'éthanol on ajoute 20 g (0,1 mole) de diphénylméthanethiol, puis verse goutte à goutte à reflux, en une heure, 25 g (0,125 mole) de bromoacétaldéhyde diéthylacétal. Après 2 heures à reflux, on évapore l'alcool sous vide, reprend à l'éther, lave à l'eau 3 fois, sèche et filtre sur charbon. En évaporant l'éther sous vide on obtient quantitativement le diéthylacétal du 2-(benzhydrylthio)acétaldéhyde.

Celui-ci est hydrolysé en chauffant 2 heures au bain-marie avec 250 ml de $H_2SO_4$ à 10%, extrait à l'éther, lavé à l'eau, séché, évaporé, cristallisé dans l'éther de pétrole, recristallisé dans l'éther isopropylique. On obtient le 2-(benzhydrylthio)acétaldéhyde (F. = 57-58°C) avec un rendement de 56%.

b) Préparation de la 2-(benzhydrylthio)acétaldoxime.

4,2 g (0,06 mole) de chlorhydrate d'hydroxylamine en solution dans 15 ml d'eau sont partiellement neutralisés avec 3,2 g (0,04 mole) de bicarbonate de sodium. On ajoute rapidement à 20°C, 9,6 g (0,04 mole) de 2-(benzhydrylthio)acétaldéhyde en solution dans 50 ml de méthanol. On agite cette solution pendant 2 heures, le pH baisse rapidement de 10 vers 5 et l'oxime précipite. On ajoute 20 ml d'eau, laisse ne contact une nuit et essore. On lave à l'eau, sèche et recristallise dans le méthanol à 80%. On obtient le composé avec un rendement global de 44%.

C'est une poudre blanche soluble dans l'éther, l'acétate d'éthyle, les alcools méthylique et éthylique.

Insoluble dans l'eau et l'éther de pétrole.

Le mélange équimoléculaire des formes "syn" et "anti" présente un point de fusion à 86-87°C.

On donnera ci-après des résultats des études pharmacologiques mettant en évidence les propriétés des composés.

Etude neuropsychopharmacologique

1)Etude du CRL 41334

Le composé CRL 41 334, en suspension dans une solution de gomme arabique, a été administré par voie intrapéritonéale sous un volume de 20 ml/kg chez la souris (mâle, NMRI, C.E.R. Janvier) et de 5 ml/kg chez le rat (mâle, $CD_1$; SPRAGUE DAWLEY, Charles River).

I - PRETOXICITE ( 3 souris par dose)

- 128 mg/kg : démarche titubante, exophtalmie, sédation, puis excitation 2 heures après l'injection.
- 256 mg/kg - démarche titubante, exophtalmie, convulsions (3/3) à 15 minutes, sédation suivie d'excitation 2 heures après l'injection. Pas de mortalité.
- 512 mg/kg - démarche titubante, exophtalmie, convulsions (3/3) pendant les 10 premières minutes. Mortalité (3/3) (1/3 en 15 minutes et 2/3 en 24 heures).

II - COMPORTEMENT GLOBAL ET REACTIVITES

Des lots de 3 animaux sont observés avant, puis 15 mn, 30 mn, 1h, 2h, 3h et 24 heures après l'administration du composé CRL 41 334.

1 - Souris

- 2, 8 et 32 mg/kg - pas de modifications nettes du comportement et des réactivités.
- 128 mg/kg - sédation pendant 30 minutes accompagnée de démarche titubante, diminution de la fréquence respiratoire, des réactivités, de la réaction de peur, du tonus musculaire. Hypothermie (-4,1° à 30 minutes, durée 1 heure).

2 - Rat

- 1, 4 et 16 mg/kg comportement, réactivités, variation du diamètre pupillaire et de la température rectale comparables à ceux du lot témoin.
- 64 mg/kg - sédation pendant 15 minutes accompagnées de dyspnée, de diminution de réactivité au toucher et du tonus musculaire
- cette sédation est suivie par une augmentation de la réaction de peur (30 minutes), de la réactivité au toucher et du tonus musculaire pendant 3 heures.
- mydriase très modérée et brève, à 1 heure.

III - RECHERCHE DE MOUVEMENTS STEREOTYPES

Des lots de 6 rats reçoivent une injection du composé CRL 41 334 ou d'amphétamine, puis sont immédiatement placés dans des boîtes de petites dimensions (20 x 10 x 10 cm) où leur comportement stéréotypeé est coté de 0 à 3 jusqu'à extinction de l'effet.

Aux deux plus fortes doses utilisées (64 et 128 mg/kg), le composé CRL 41 334 provoque des stéréotypes nettes dont l'intensité est comparable à celle obtenue respectivement avec 1 et 2 mg d'amphétamine.

Cependant, les cinétiques d'action sont différentes. Les stéréotypies induites par 2 mg/kg d'amphétamine atteignent leur maximum en 60 à 90 minutes et durent 270 minutes, alors que les stéréotypies induites par 128 mg/kg du composé CRL 41 334 atteignent leur maximum en 120-150 minutes et durent plus de 420 minutes.

IV - INTERACTION AVEC L'APOMORPHINE

1 - Souris

Des lots de 6 souris reçoivent le composé CRL 41 334 une demi-heure avant l'injection sous-cutanée d'apomorphine, à la dose de 1 ou 16 mg/kg.

a) Apomorphine 1 mg/kg
b) Apomorphine 16 mg/kg

A la plus forte dose utilisée (128 mg/kg) le composé CRL 41 334, seul, entraîne une hypothermie et aggrave l'hypothermie induite par l'apomorphine (1 mg/kg).

Les comportements de verticalisation et de stereotypies ne sont pas modies.

2 - Rat

Le composé CRL 41 334 est administré à des lots de 6 rats une demi-heure avant l'injection sous-cutanée de 0,5 mg/kg d'apomorphine.

Aux deux plus fortes does utilisés (16 et 64 mg/kg), le composé CRL 41 334 potentialise les stéréotypies induites par l'apomorphine.

V - INTERACTION AVEC L'AMPHETAMINE

L'amphétamine (2 mg/kg) est injectée par voie intrapéritonéale à des lots de 6 rats, une demi-heure après l'administration du composé CRL 41 334.

Aux doses de 16, mais surtout 64 mg/kg, Le composé CRL 41 334 potentialise les stéréotypies amphétaminiques.

VI - INTERFRACTION AVEC LA RESERPINE

Quatre heures après l'injection intrapéritonéale de 2,5 mg/kg de réserpine, des lots de 12 souris reçoivent le composé CRL 41 334.

1 - Action sur la température
2 - Action sur le ptosis

A la plus forte dose utilisée (128mg/kg), Le composé CRL 41 334 aggrave modérément l'hypothermie réserpinique. Le ptosis, déjà maximal, n'est pas modifié.
A noter qu'au temps 24 heures, le composé CRL 41 334 (8, 32 ou 128 mg/kg) semble s'opposer à l'hypothermie et au ptosis réserpiniques.

VII - INTERACTION AVEC L'OXOTREMORINE

Le composé CRL 41 334 est administré à des lots de 6 souris une demi-heure avant l'injection intrapéritonéale de 0,5 mg/kg d'oxotrémorine.

1 - Action sur la température

A la plus forte dose utilisée (128 mg/kg), le composé CRL 41 334 entraîne une hypothermie et aggrave l'hypothermie induite par l'oxotrémorine.

2 - Action sur les tremblements

Les tremblements dus à l'oxotrémorine ne sont pas modifiés par le composé CRL 41 334.

3 - Action sur les symptômes cholinergiques périphériques

A la dose de 128 mg/kg, le composé CRL 41 334 diminue la défécation sans modifier la salivation et la lacrymation.

VIII - ACTION SUR LE TEST DES QUATRES PLAQUES, LA TRACTION ET L'ELECTROCHOC

Le test est pratiqué sur des lots de 10 souris, une demi-heure après l'administration du composé CRL 41 334.
Le composé CRL 41 334 ne modifie pas nettement le nombre de passages punis. Il ne provoque pas d'incapacité motrice majeure.
A la plus forte dose utilisée (128 mg/kg), le composé CRL 41 334 s'oppose faiblement aux effets convulsivants sans modifier les effets létaux de l'électrochoc.

IX - ACTION SUR LA MOTILITE SPONTANEE

Après avoir reçu le composé CRL 41 334, les souris sont placées en actimètre où leur motilité est enregistrée pendant 30 minutes.

1) Composé administré 30 minutes avant passage dans l'actimètre

Aux doses de 32 et 64 mg/kg, le composé CRL 41 334 entraîne une augmentation de la motilité spontanée de la souris; à la dose de 128 mg/kg, cet effet n'apparaît plus.

2) Composé administré 2 heures avant passage dans l'actimètre

Aux doses de 8, mais surtout 32 et 128 mg/kg, le composé CRL 41 334 entraîne une augmentation de la motilité spontanée de la souris.

X - ACTION SUR L'AGRESSIVITE INTERGROUPES

Après avoir séjourné pendant 3 semaines dans chacune des moitiés d'une cage séparées par une cloison opaque, des groupes de 3 souris reçoivent le composé CRL 41 334. Une demi-heure plus tard, les deux groupes d'une même cage sont réunis par retrait de la cloison et on note le nombre de combats qui surviennent en 10 minutes. Le test est pratiqué pour moitié sur les souris habituelles (NMRI, C.E.R. Janvier) et pour moitié sur des souris NMRI (Iffa Credo).
Aux deux doses utilisées (32 et 128 mg/kg), le composé CRL 41 334 diminue le nombre de combats. -

XI - ACTION VIS-A-VIS DE QUELQUES COMPORTEMENTS PERTURBES PAR DIVERS AGENTS

1 - Motilité réduite par habituation à l'enceinte

Après 18 heures de séjour dans les actimètres, les souris (6 par dose, 12 témoins) reçoivent le composé CRL 41 334. Elles sont aussitôt replacées dans leurs enceintes respectives et, une demi-heure plus tard, on enregistre leur motilité pendant 30 minutes.

Dès la dose de 2 mg/kg, le composé CRL 41 334 entraîne une reprise nette de l'activité motrice chez la souris hatituée à son enceinte.

2 - Motilité réduite par agression hypoxique

Une demi-heure après avoir reçu le composé CRL 41 334, les souris (10 par dose, 20 témoins) sont soumises à une anoxie hypobare aigüe (dépression de 60 mmHg en 90 secondes, détente de 45 secondes), puis elles sont placées en actimètre où leur motilité est enregistrée pendant 10 minutes.

Dès la dose de 2mg/kg, le composé CRL 41 334 entraîne une amélioration de la récupération motrice chez la souris dont la motilité a été déprimée à la suite d'un séjour bref dans une enceinte à pression réduite. Cette amélioration est importante pour les deux plus fortes doses utilisées (32 et 128 mg/kg).

3 - Anoxie asphyxique

Des lots de 20 souris reçoivent le composé CRL 41 334 une demi-heure avant l'administration intrapéritonéale de 32 mg/kg de triiodoéthylate de gallamine.

A la plus forte dose utilisée (128 mg/kg), le composé CRL 41 334 augmente le délai d'apparition de la mort consécutive à une anoxie provoquée par un curarisant. Le délai d'apparition des convulsions n'est pas modifié.

XII - INTERACTION AVEC LE BARBITAL

Une demi-heure après l'administration du composé CRL 41 334, des lots de 20 souris reçoivent une injection intrapéritonéale de barbital (220 mg/kg).

Aux doses de 8, 32 et 128mg/kg, le composé CRL 41 334 entraîne une diminution importante de la durée du sommeil barbiturique.

XIII - ACTION SUR LE "DESESPOIR COMPORTEMENTAL"

Une demi-heure après avoir reçu le composé CRL 41 334, des lots de 6 souris (mâles, $CD_1$, Charles River) sont placés dans un bécher rempli d'eau sur une hauteur de 6 cm. On note la durée totale d'immobilité entre la 2ème et la 6ème minutes suivant l'immersion.

Aux doses de 8, 32 et 128 mg/kg, le composé CRL 41 334 diminue la durée de l'immobilité de la souris placée en immersion forcée.

XIV - CONCLUSION

L'étude neuropsychopharmacologique du composé CRL 41 334 montre :

- un effet stimulant chez la souris : hypermotilité, reprise nette de l'activité motrice chez la souris habituée à son enceinte, amélioration de la récupération motrice après hypoxie, antagonisme du sommeil barbiturique et diminution de l'hypomotilité de "désespoir".

A forte dose, on note un effet sédatif bref: sédation, hypo-réactivité, hypo-agressivité, hypothermie, aggravation des hypothermies induites par la réserpine et l'oxotrémorine, diminution modérée de l'effet convulsivant de l'électrochoc et du délai d'apparition de la mort consécutive à une anoxie provoquée par un curarisant. Deux heures après administration du produit, cet effet sédatif laisse place à l'effet stimulant.

De plus, chez le rat, on note à forte dose : des stéréotypies et une potentialisation des stéréotypies induites par l'apomorphine et l'amphétamine.

Cet effet stimulant ne semble se développer qu'après une période de latence importante de l'ordre de 2 heures au moins.

2) Etude du CRL 41 055

Le CRL 41 055, en suspension dans une solution de gomme arabique, a été administré par voie intrapéritonéale sous un volume de 20 ml/kg chez la souris (mâle, NMRI, Evic Ceba).

Prétoxicite (3 souris par dose)

- 64 mg/kg : excitation, stéréotypies (reniflements, redressements) apparaîssant après 45 minutes et durant plus de 2 heures.
- 128 et 256 mg/kg : pendant 45 minutes sédation, fréquence respiratoire diminuée, contractions abdominales suivies d'excitation, stéréotypies (reniflements, redressements) pendant plus de 2 heures.

Pas de mortalité.
- 512 mg/kg : pendant 45 minutes sédation, fréquence respiratoire diminuée, contractions abdominales, titubations subconvulsives suivies d'excitation, stéréotypies (moins nettes qu'aux doses plus faibles).
. 18 heures après l'injection, convulsions intermittentes présentes chez toutes les souris, entrecoupées (2/3) de stéréotypies (toilette, mâchonnements).
. mortalité (1/3) après 48 heures.
- 1024 mg/kg : mêmes symptômes.

Comportement global et réactivités

Des lots de 3 souris sont observés, puis 15 mn, 30 mn, 1 h, 2 h, 3 h et 24 heures après l'administration de CRL 41 055.
- 2 mg/kg : pas de modifications nettes du comportement et des réactivités.
- 8 et 32 mg/kg : excitation pendant 2 heures.
- 128 mg/kg : sédation pendant 30 minutes faisant place à une excitation tardive (2 heures) durant 1 heure avec augmentation de la réactivité au toucher et de la réaction de peur.

Action sur la motilité spontanée

Une demi-heure après avoir reçu le CRL 41 055, les souris (6 par dose, 12 témoins) sont placées en actimètre où leur motilité est enregistrée pendant 30 minutes.
le CRL 41 055 entraîne aux doses de 32 et 128 mg/kg une augmentation de motilité spontanée chez la souris.

Motilité réduite par habituation à l'enceinte

Après 18 heures de séjour dans les actimètres, les souris (6 par dose, 12 émoins) reçoivent le CRL 41 055. Elles sont aussitôt replacées dans leurs enceintes respectives et, une demi-heure plus tard, on enregistre leur motilité pendant 30 minutes.
Le CRL 41 055, aux doses de 32 et 128 mg/kg, provoque une reprise nette de l'activité motrice chez la souris habituée à son enceinte.

Conclusion

Le CRL 41 055 présente, chez la souris un effet stimulant : excitation, mouvements d'apparence stéréotypés, hypermotilité et reprise nette de l'activité motrice chez la souris habituée à son enceinte. Cette stimulation est parfois précédée par une phase de sédation.
Le CRL 41 055 s'est avéré utile en thérapeutique humaine comme antidépresseur, à la dose de trois gélules de 50 mg par jour.

3) Etude du CRL 40 956

Le CRL 40 956, en suspension dans une solution de gomme arabique a été administré par voie intrapéritonéale sous un volume de 20 ml/kg chez la souris (mâle, NMRI, Evic Céba) et de 5 ml/kg chez le rat (mâle, $CD_1$, Sprague Dawley, Charles River).

Prétoxicité ( 3 souris par dose)

- 1024 et 512 mg/kg : crampes abdominales, dyspnée, pas de mortalité.
- 256 mg/kg : mêmes symptômes.

Comportement global et réactivités

Des lots de 3 animaux sont observés avant, puis 15 mn, 30 mn, 1 h, 2 h, 3 h et 24 heures après l'administration de CRL 40 956.

1 - Souris

- 512 mg/kg : sédation pendant 2 à 3 heures avec diminution de la réactivité au toucher et au tonus musculaire pendant 1 à 3 heures.
hypothermie (-2°9) pendant 3 heures.
- 128, 32 et 8 mg/kg : pas de symptômes nets.

2 - Rat

- 256 mg/kg : sédation pendant 3 heures avec diminution de la réactivité au toucher et du tonus musculaire.
- 64 mg/kg : sédation pendant 3 heures avec diminution
- 16 et 4 mg/kg : comportement et réactivités comparables à deux du lot témoin.

Interaction avec l'apomorphine

Le CRL 40 956 est administré à des lots de 6 rats une demi-heure avant l'injection sous cutanée de 0,5 mg/kg d'apomorphine.

Aux doses de 64 et 256 mg/kg le CRL 40 956 diminue très modérément l'intensité des stéréotypies induites par l'apomorphine.

Action sur la motilité spontanée

Une demi-heure après avoir reçu le CRL 40 956, les souris (6 par dose, 12 témoins) sont placées en actimètre ou leur motilité est enregistrée pendant 30 minutes.

A la forte dose (512 mg/kg) le CRL 40 956 diminue fortement l'activité motrice spontanée de la souris.

Action sur l'agressivité intergroupes

Après avoir séjourné pendant 3 semaines dans chacune des moitiés d'une cage séparée par une cloison opaque, des groupes de 3 souris reçoivent le CRL 40 956. Une demi-heure plus tard, les deux groupes d'une même cage sont réunis par retrait de la cloison et on note le nombre de combats qui surviennent en 10 minutes.

A la dose de 128 mg/kg le CRL 40 956 diminue le nombre de combats.

Action vis-à-vis de l'anoxie asphyxique

Des lots de 10 souris reçoivent le CRL 40 956 une demi-heure avant l'administration intrapéritonéale de 32 mg/kg de triodoéthylate de Gallamine.

A forte dose (512 mg/kg) le CRL 40 956 retarde modérément l'apparition des convulsions et de la mort consécutive à une anoxie asphyxique provoquée par un curarisant.

Interaction avec le barbital

Une demi-heure après l'administration du CRL 40 956 des lots de 10 souris reçoivent une injection intrapéritonéale de 200 mg/kg de barbital.

A la forte dose (512 mg/kg) le CRL 40 956 diminue la durée du sommeil barbiturique.

Action sur le "désespoir comportemental

Une demi-heure après avoir reçu le CRL 40 956, des lots de 10 souris (mâles $CD_1$ Charles River) sont plongées dans une enceinte réduite remplie d'eau sur une hauteur de 6 cm. On note la durée totale d'immobilité entre la 2ème et la 6ème minute suivant l'immersion.

A la forte dose (512 mg/kg) le CRL 40 956 diminue la durée d'immobilité "de désespoir".

En conclusion, le CRL 40 956 s'avère être un sédatif.

ETUDE IMMUNOLOGIQUE

Réaction d'hypersensibilité retardée aux globules rouges de mouton

Selon la méthode de Miller, Lagrange et Mackaness ("Immunopotentiation with BCG II modulation of the response to sheep red blood cells", Journal of the National Cancer Institute 1973, 51, 5, 1669 - 1676).

Le composé à tester est administré par voie orale a des souris femelles de souche $OF_1$ trois jours avant immunisation.

Les résultats sont exprimés en pourcentage d'augmentation du coussinet plantaire.

Composé CRL 41 334

| Exp | % d'augmentation du cousinet (mm) | Doses du produit en mg/kg 0 | 0,1 | 1 | 10 | 100 |
|---|---|---|---|---|---|---|
| 1 | m | 9,2 | 10,6 | 10,2 | 12,0 | 14,7x |
| | ± e.s.m. | ± 1,06 | ± 1,42 | ± 1,22 | ± 2,16 | ± 2,93 |
| | Ind. Activité | | 1,15 | 1,10 | 1,29 | 1,59 |
| 2 | m | 8,4 | 11,0 | 10,5 | 11,2 | 13,1 |
| | ± e.s.m. | ± 1,35 | ± 1,77 | ± 1,75 | ± 1,67 | ± 1,92 |
| | Ind. Activité | | 1,31 | 1,25 | 1,34 | 1,56 |

Composé CRL 41 335

| Exp | % d'augmentation du cousinet (mm) | Doses du produit en mg/kg 0 | 0,1 | 1 | 10 | 100 |
|---|---|---|---|---|---|---|
| 1 | m | 8,9 | 11,5 | 8,4 | 8,9 | 6,9 |
| | ± e.s.m. | ± 0,92 | ± 1,75 | ± 0,63 | ± 2,41 | ± 1,42 |
| | Ind. Activité | | 1,29 | 0,94 | 1,00 | 0,77 |

Il apparaît ainsi en particulier que le composé CRL 41 334 se présente comme un produit ayant d'une part une action anti-dépressive au niveau du système nerveux central et d'autre part une action immunostimulante, action qui est d'autant plus inattendue que le composé CRL 41 335 est dépourvu d'une telle action immostimulante.

Le composé CRL 41 334 administré par voie orale à raison de 4 fois 50 mg par jour a donné de bons résultats chez l'homme dans le traitement des dépressions des brûlés.

Les compositions thérapeutiques selon l'invention peuvent être administrées à l'homme ou aux animaux par voie orale et rectale.

Elles peuvent être sous la forme de prépa rations solides ou semi-solides. Comme exemple, on peut citer les comprimés, les gélules, les suppositoires, ainsi que les formes-retard.

Dans ces compositions, le principe actif est généralement mélangé avec un ou plusieurs excipients pharmaceutiquement acceptables habituels bien connus de l'homme de l'art.

La quantité de principe actif administrée dépend évidemment du patient qui est traité, de la voie d'administration et de la sévérité de la maladie.

**Revendications pour les états contractants BE, CH, DE, FR, GD, IT, LI, LU, NL, SE**

1. Compositions thérapeutiques contenant à titre de principe actif un dérivé du benzhydrylthiométhane de formule

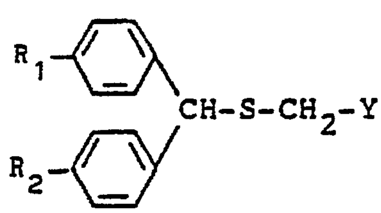

II

dans laquelle Y représente un groupe $-CONH_2$, $-CO-NHOH$ ou $-CH=N-OH$, et $R_1$ et $R_2$ représentent indépendamment l'un de l'autre un atome d'hydrogène ou de fluor.

2. Composition thérapeutique selon la revendication 1, contenant à titre de principe actif le 2-(4,4'-difluoro benzhydrylthio)-acétamide.

3. Composition thérapeutique selon la revendication 1, contenant à titre de principeactif le 2-(benzhydrylthio)acétamide.

4. Composition thérapeutique selon la revendication 1, contenant à titre de principe actif la 2-(benzhydrylthio)acétaldoxime.

**Revendications pour les états contractants AT, GR, ES**

1. Procédé de préparation d'une composition thérapeutique, caractérisé en ce que l'on met un dérivé du benzhydrylthiométhane de formule

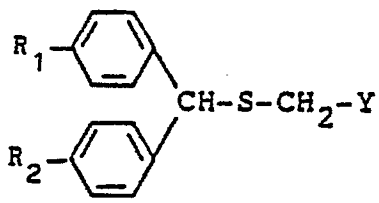

II

dans laquelle Y représente un groupe $-CONH_2$, $-CO\text{-}NHOH$ ou $-CH=N\text{-}OH$
et $R_1$ et $R_2$ représentent indépendamment l'un de l'autre un atome d'hydrogène ou de fluor sous une forme pharmaceutiquement acceptable.

2. Procédé selon la revendication 1, caractérisé en ce que l'on met le 2-(4,4'-difluoro benzhydrylthio)-acétamide sous une forme pharmaceutiquement acceptable.

3. Procédé selon la revendication 1, caractérisé en ce que l'on met le 2-(benzhydrylthio)acétamide sous une forme pharmaceutiquement acceptable.

4. Procédé selon la revendication 1, caractérisé en ce que l'on met la 2-(benzhydrylthio)acétaldoxime sous une forme pharmaceutiquement acceptable.

**Claims for the Contracting States BE, CH, DE, FR, GB, IT, LI, LU, NL, SE**

1. Therapeutic compositions containing as active principle a derivative of benzhydrylthiomethane having the Formula:

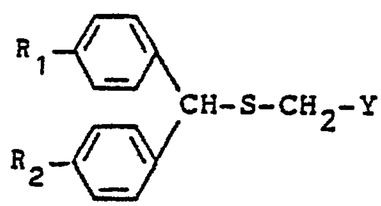

II

where Y denotes a $-CONH_2$, $-CO\text{-}NHOH$ or $-CH=N\text{-}OH$ group, and $R_1$ and $R_2$ denote a hydrogen or fluorine atom independently of one another.

2. A therapeutic composition according to claim 1, containing as active principle 2-(4,4'-difluorobenzhydrylthio) acetamide.

3. A therapeutic composition according to claim 1, containing as active principle 2-(benzhydrylthio) acetamide.

4. A therapeutic composition according to claim 1, containing as acitve principle 2-(benzhydrylthio) acetaldoxime.

**Claims for the Contracting States AT, GR, SP**

1. A process for the preparation of a therapeutic composition, characterized in that a pharmaceutically acceptable form is given to a derivative of benzhydrylthiomethane having the Formula:

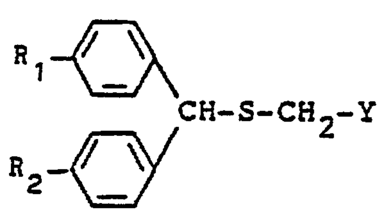

II

where Y denotes a $-CONH_2$, $-CO\text{–}NHOH$ or $-CH=N\text{–}OH$ group, and $R_1$ and $R_2$ denote a hydrogen or fluorine atom independently of one another.

2. A process according to claim 1, characterized in that a pharmaceutically acceptable form is given to 2-(4,4'-difluorobenzhydrylthio) acetamide.

3. A process according to claim 1, characterized in that a pharmaceutically acceptable form is given to 2-(benzhydrylthio) acetamide.

4. A process according to claim 1, characterized in that a pharmaceutically acceptable form is given to 2-(benzhydrylthio) acetaldoxime.

**Patentansprüche für die Vertragsstaaten BE, CH, DE, FR, GB, IT, LI, LU, NL, SE**

1. Therapetische Zusammensetzung enthaltend als Wirkstoff ein Benzylhydrothiomethan-Derivat der Formel

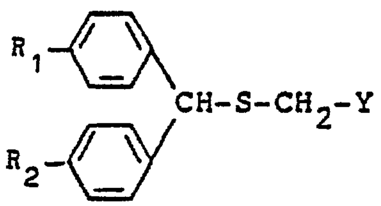

II

worin Y eine Gruppe der Formel $-CONH_2$, $-CO-NHOH$ oder $-CH=N-OH$ und $R_1$ und $R_2$ voneinander unabhängig Wasserstoff- oder Fluoratome bedeuten.
2. Therapeutische Zusammensetzung nach Anspruch 1, dadurch gekennzeichnet, daß sie als Wirkstoff 2-(4,4′-Difluorbenzhydrylthio)-acetamid enthält.
3. Therapeutische Zusammensetzung nach Anspruch 1, dadurch gekennzeichnet, daß sie als Wirkstoff 2-(Benzyhydrylthio)-acetamid enthält.
4. Therapeutische Zusammensetzung, dadurch gekennzeichnet, daß sie als Wirkstoff 2-(Benzhydrylthio)-acetaldoxim enthält.

**Patentansprüche für die Vertragsstaaten AT, GR, ES**

1. Verfahren zur Herstellung einer therapeutischen Zusammensetzung, dadurch gekennzeichnet, daß man ein Benzylhydrothiomethan-Derivat der Formel

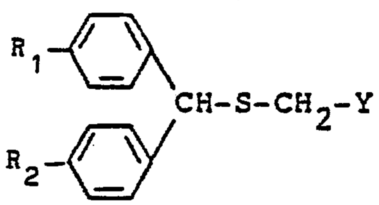

II

worin Y eine Gruppe der Formel $-CONH_2$, $-CO-NHOH$ oder $-CH=N-OH$ und $R_1$ und $R_2$ voneinander unabhängig Wasserstoff- oder Fluoratome bedeuten, in eine pharmazeutisch annehmbare Form bringt.
2. Verfahren nach Anspruch 1, dadurch gekennzeichnet, daß man 2-(4,4′-Difluorbenzhydrylthio)-acetamid in eine pharmazeutisch annehmbare Form bringt.
3. Verfahren nach Anspruch 1, dadurch gekennzeichnet, daß man 2-(Benzhydrylthio)-acetamid in eine pharmazeutisch annehmbare Form bringt.
4. Verfahren nach Anspruch 1, dadurch gekennzeichnet, daß man 2-(Benzhydrylthio)-acetaldoxim in eine pharmazeutisch annehmbare Form bringt.